# EUROPÄISCHE PATENTSCHRIFT

(11) **EP 0 368 358 B1**
(45) Veröffentlichungstag und Bekanntmachung des Hinweises auf die Patenterteilung: **10.08.1994**
(21) Anmeldenummer: 89120918.1
(22) Anmeldetag: 10.11.1989
(51) Int. Cl.: G01N 31/22

(54) **Verfahren und Messgerät zur kontinuierlichen Messung von Quecksilber und anderen Schwermetallen in Abgasen**
Method and apparatus for the continuous measuring of mercury and other heavy metals in waste gases
Méthode et appareil de mesure continue du mercure et autres métaux lourds dans les gaz résiduaires

(30) Priorität: 10.11.1988 DE 3838193
(43) Veröffentlichungstag der Anmeldung: 16.05.1990
(73) Patentinhaber: GSB Gesellschaft zur Beseitigung von Sondermüll in Bayern mbH, D-80797 München (DE)
(72) Erfinder: Luxenberg, Peter, Dr.rer.nat., D-8000 München 40 (DE)
(74) Vertreter: Tetzner, Volkmar, Dr.-Ing. Dr. jur.

(56) Entgegenhaltungen:
- EP-A- 0 012 221
- US-A- 3 434 800
- US-A- 4 309 186
- CHEMICAL ABSTRACTS, Band 108, Nr. 20, 16. Mai 1988, Seite 342, Spalte 2,Zusammenfassung Nr. 172822j, Columbus, Ohio, US; & JP-A-63 26 556

## Beschreibung

Die Erfindung betrifft ein Verfahren (entsprechend dem Oberbegriff des Anspruches 1) sowie ein Meßgerät (gemäß dem Gattungsbegriff des Anspruches 6) zur kontinuierlichen Messung von Schwermetallen in Abgasen, insbesondere von Müllverbrennungsanlagen.

Das den Müllverbrennungsanlagen zugeführte Material enthält zeitweise erhebliche Mengen an Quecksilber und anderen Schwermetallen, die auch mit einer sehr intensiven Rauchgasreinigung nicht abgefangen werden und daher von vornherein ausgesondert werden müssen. Diese Aussonderung ist jedoch aufgrund der Inhomogenität insbesondere von Sondermüll und wegen der im Verhältnis zu den Abfallmengen äußerst geringen Quecksilbermenge ohne vorherige Einkreisung unmöglich.

Es ist daher anzustreben, über eine lückenlose und kontinuierliche Erfassung der in den Abgasen, insbesondere von Müllverbrennungsanlagen, enthaltenen Emissionen von Quecksilber und anderen Schwermetallen auch deren Herkunft einzukreisen.

Durch "Chemical Abstracts, Vol.108, Ref.No.20, Ref. 172822j" ist bereits ein Verfahren entsprechend dem Oberbegriff des Anspruches 1 bekannt, bei dem zur Messung von Quecksilber in Abgasen ein Probengasstrom auf 600°C erhitzt und dann mit einer HCl-SnCl₂ -Lösung als Reduktionsmittel behandelt wird. Anschließend erfolgt eine Kühlung des Gas-Flüssigkeitsgemisches und eine Wäsche mit einer HCl-SnCl₂-Lösung. Nach Trocknung der Gasprobe wird eine Absorptionsmessung durchgeführt.

Mit diesem bekannten Verfahren lassen sich zwar Quecksilber (Hg), nicht jedoch andere Schwermetalle, wie Arsen (As), Antimon (Sb) und Wismut (Bi), bestimmen. Nachteilig ist bei dem bekannten Verfahren ferner die komplizierte Eichung eines nach diesem Verfahren arbeitenden Gerätes. Hierfür müßte vor der HCl-SnCl₂-Dosierung eine gesonderte Dosierstelle vorgesehen werden.

Der Erfindung liegt daher die Aufgabe zugrunde, ein Verfahren (entsprechend dem Oberbegriff des Ansprüches 1) sowie ein Meßgerät (gemäß dem Gattungsbegriff des Ansprüches 6) zu entwickeln, das neben Quecksilber auch die Bestimmung anderer Schwermetalle, wie insbesondere Arsen, Antimon und Wismut, ermöglicht und das darüber hinaus eine einfache Eichung gestattet.

Diese Aufgabe wird erfindungsgemäß durch die kennzeichnenden Merkmale der Ansprüche 1 und 6 gelöst. Zweckmäßige Ausgestaltungen der Erfindung sind Gegenstand der Unteransprüche.

Quecksilber und andere Schwermetalle, wie insbesondere Arsen, Antimon und Wismut, können in den Abgasen (beispielsweise in den Rauchgasen einer Müllverbrennungsanlage) elementar oder als Verbindung, ferner gasförmig oder an Staub gebunden, vorliegen. Um alle Schwermetalle zu erfassen, muß das Abgas ungefiltert auf kürzestem Wege einem Reaktor zugeführt werden, in dem die Gasaufbereitung erfolgt.

Zu diesem Zweck wird ein aus dem Abgas abgezweigter Probengasstrom in einem Ofen auf eine Temperatur von mindestens 700°C, vorzugsweise auf etwa 800°C, erhitzt.

Hierdurch werden restliche, im Abgas noch vorhandene organische Substanzen verbrannt. Schwermetalle (vor allem diejenigen, die aufgrund ihrer leichten Flüchtigkeit emissionsrelevant sind) werden freigesetzt. Schwermetallverbindungen werden zersetzt.

Bei hohen Anteilen an unverbrannten organischen Verbindungen oder bei hohen Anteilen von SO₂ kann der Ofen mit einem Katalysator, beispielsweise Platin-Asbest, gefüllt werden.

Dem erhitzten Probengasstrom wird sodann ein flüssiges saures Medium zugeführt, insbesondere Salzsäure und/oder Salpetersäure in einer Konzentration von 0,1 bis 5n, vorzugsweise 1n. Die Vermischung des erhitzten Probengasstromes mit dem flüssigen sauren Medium erfolgt auf kürzestem Wege, damit die im Ofen freigesetzten elementaren Schwermetalle sich möglichst wenig wieder an Staub anlagern oder Verbindungen bilden können.

Die hohe Temperatur begünstigt einen schnellen Stoffaustausch zwischen dem Gas und der Flüssigkeit. Das Gas nimmt hierbei Säure und Wasser auf, die in der nachfolgenden Reaktionsschlange wieder auskondensieren und somit ebenfalls zur Reinigung der Gase und zur Überführung von Schwermetallverbindungen in Säure beitragen.

Der mit dem flüssigen sauren Medium vermischte erhitzte Probengasstrom durchsetzt sodann eine erste Reaktionsschlange, die auf einer Temperatur zwischen 50 und 100°C, vorzugsweise etwa 70°C, gehalten wird. Die genannte Temperatur gewährleistet einen guten Stoffaustausch zwischen dem Gas und dem flüssigen sauren Medium.

Wenn der HCl-Gehalt des Abgases kleiner als etwa 20 mg/Nm³ beträgt, kann als saures Medium Salpetersäure verwendet werden. Salpetersäure ist frei von Quecksilber. Mit dem restlichen HCl des Abgases sich bildendes NOCl/Cl₂, das die Quecksilbermessung stört, wird bei der folgenden Reduktionsreaktion wieder entfernt.

Bei höheren Konzentrationen von HCl im Rauchgas ist Salzsäure vorzuziehen, da anderenfalls die nachfolgende Reaktionsstrecke unnötig verlängert werden muß, um alles gebildete NOCl/Cl₂ wieder zu beseitigen. Cl₂ reagiert auch bei Umgebungstemperatur mit elementaren Schwermetallen. Die Reaktion findet sowohl in der Gasphase statt, als auch an fast allen Materialien, die Cl₂ stark absorbieren. Beispielsweise absorbieren PVC/Silikonschläuche, die für einige Minuten mit Cl₂ beaufschlagt werden, mehrere Stunden lang die meisten hier interessierenden Schwermetalle im fraglichen Meßbereich.

Erfindungsgemäß werden daher alle mit dem Gas in Berührung kommenden Teile des Meßgerätes aus Quarzglas oder aus PTFE hergestellt. Bei PTFE werden zweckmäßig besonders dichte Sorten verwendet, die im interessierenden Meßbereich keine merkliche Absorption für Hg⁰ aufweisen.

Nach dem Durchsetzen der ersten Reaktionsschlange (in der die Reaktion zwischen Gas und Säure erfolgt) wird als Reduktionsmittel ein Tetrahydroboranat, vorzugsweise NaBH₄, zugesetzt. Das Reaktionsgemisch wird dann durch eine zweite Reaktionsschlange geleitet, die auf einer Temperatur unterhalb Raumtemperatur, vorzugsweise zwischen 5 und 10°C, gehalten wird. Die zweite Reaktionsschlange ist so dimensioniert, daß die gelösten Schwermetalle, wie Hg, As, Sb und Bi, in elementare Schwermetalle bzw. in die Hydride umgewandelt und ausgetrieben werden. Bei der genannten Temperatur wird das Gas einerseits ausreichend getrocknet, andererseits ist der Dampfdruck der Schwermetalle (im interessierenden Meßbereich) noch bei weitem ausreichend, um Verluste durch Kondensation zu vermeiden.

Mit der beschriebenen Zugabe von Säure und - anschließend - Tetrahydroboranat als Reduktionsmittel lassen sich neben Quecksilber auch Arsen, Antimon und Wismut bestimmen. Letztere werden durch das Tetrahydroboranat (z.B. NaBH₄) in die unter diesen Bedingungen ebenfalls gasförmigen Hydride (AsH₃, SbH₃, BiH₃,) überführt und können als solche im selben Gasstrom wie Hg mittels handelsüblicher Detektoren als Summe oder einzeln bestimmt werden. Bei dem eingangs beschriebenen bekannten Verfahren, das SnCl₂ als Reduktionsmittel einsetzt, ist dagegen die Bildung der Hydride von As, Sb und Bi nicht möglich.

Die beim erfindungsgemäßen Verfahren (im Unterschied zum geschilderten bekannten Stand der Technik) vorgenommene getrennte Zugabe von Säure und Reduktionsmittel ermöglicht eine einfache Eichung des Gerätes. Hierzu braucht statt Rauchgas nur Luft durch die Apparatur gesaugt und die reine Säure durch eine Säure mit auf das Gas/Flüssigkeitsverhältnis abgestimmten Mengen an Hg, As, Sb und Bi ersetzt zu werden.

Nach dem Durchsetzen der zweiten Reaktionsschlange wird das mit elementaren Schwermetallen bzw. den Hydriden beladene Gas von der Flüssigkeit getrennt. Zur Vermeidung einer Wiederverdampfung von Flüssigkeit wird die Trenneinrichtung zweckmäßig gleichfalls auf einer Temperatur von 5 bis 10°C gehalten.

Das Gas wird sodann einer Analysenstufe zugeführt, in der der Gehalt des Gases an Schwermetallen nach bekannten Methoden bestimmt wird. Dies kann mittels Atomabsorption, durch Photometer, Photoyonisationsdetektor mit vorgeschalteter gaschromatographischer Säule (zur Trennung der Hydride von NO) oder mit Hilfe der Emissionsspektrometrie erfolgen.

Die Förderstrecke zum Transport des mit elementaren Schwermetallen bzw. den Hydriden beladenen Gases von der Trenneinrichtung zur Analysenstufe, die beispielsweise aus einem PTFE-Schlauch besteht, wird erfindungsgemäß zweckmäßig auf eine Temperatur erwärmt, bei der einerseits keine Kondensatbildung erfolgt und andererseits keine Reaktionen der Schwermetalle mit anderen Stoffen, insbesondere mit Sauerstoff, eintreten. Die Temperatur dieser Förderstrecke liegt zweckmäßig zwischen 20 und 60°C, vorzugsweise bei etwa 40°C.

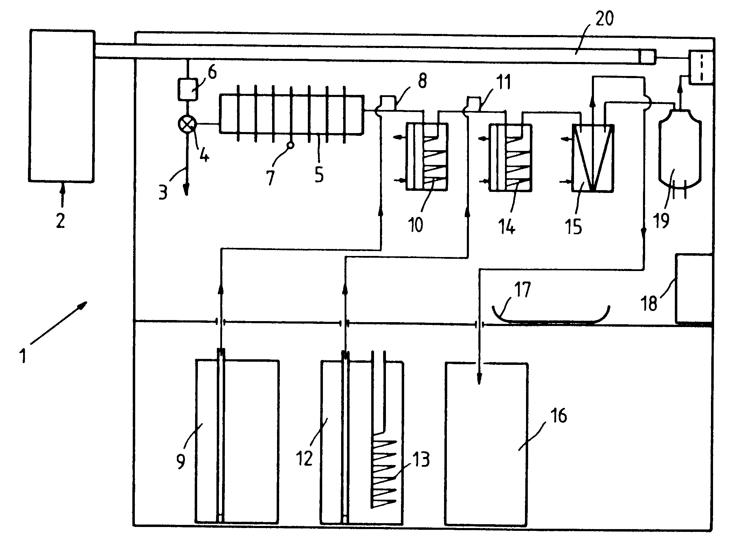

Ein Ausführungsbeispiel eines nach dem erfindungsgemäßen Verfahren arbeitenden Meßgerätes ist schematisch in der Zeichnung veranschaulicht.

Das Meßgerät besteht aus einer Einrichtung 1 zur Gasaufbereitung sowie einem Analysengerät 2.

Die Einrichtung 1 zur Gasaufbereitung enthält eine Sonde 3, über die ein Probengasstrom aus dem die Rauchgase einer Müllverbrennungsanlage führenden Kamin abgezweigt wird. Über ein Zweiwegeventil 4 gelangt der Probengasstrom in einen infrarotbeheizten Ofen 5 mit einem auswechselbaren Verbrennungsrohr aus Quarzglas.

Mittels des Zweiwegeventils 4 kann statt des Probengasstromes Luft (zum Zwecke der Nullpunktseinstellung) durch das Gerät gesaugt werden, wobei die Luft durch ein Absorptionsgefäß strömt.

Die Temperatur des Ofens 5 wird mittels eines Thermometers 7 überwacht. Der Probengasstrom wird in dem Ofen 5 auf etwa 800°C erhitzt, so daß restliche Kohlenwasserstoffe verbrennen und Schwermetalle, wie Quecksilber, Arsen, Antimon und Wismut, aus Staub und Verbindungen in gasförmige Schwermetalle überführt werden.

Im unmittelbaren Anschluß an den Ofen 5 ist eine Einrichtung 8 zur dosierten Einführung eines flüssigen sauren Mediums (insbesondere verdünnte Salzsäure oder Salpetersäure) in den Probengasstrom vorgesehen. Die der Dosiereinrichtung 8 zugeführte Säure wird einem Vorratsbehälter 9 entnommen.

Das Gas-Säuregemisch gelangt sodann in eine erste Reaktionsschlange 10, die auf eine Temperatur von ca. 70°C thermostatisiert ist.

Anschließend wird in das Gas-Flüssigkeitsgemisch mittels einer Dosiereinrichtung 11 als Reduktionsmittel ein Tetrahydroboranat, vorzugsweise NaBH₄ eingeführt, das einem Vorratsbehälter 12 entnommen wird. Der Inhalt des Vorratsbehälters 12 wird insbesondere bei sommerlichen Temperaturen zweckmäßig mittels einer Kühleinrichtung 13 gekühlt.

Das mit dem Reduktionsmittel versehene Gas-Flüssigkeitsgemisch durchsetzt sodann eine zweite Reaktionsschlange 14, die auf eine Temperatur zwischen 5 und 10°C thermostatisiert ist.

Anschließend erfolgt in einer Trenneinrichtung 15 die Trennung des Gases von der Flüssigkeit. Die Trenneinrichtung 15 ist zweckmäßig gleichfalls auf 5 bis 10°C thermostatisiert. Die in der Trenneinrichtung 15 abgeschiedene Flüssigkeit gelangt in einen Auffangbehälter 16.

Eine Auffangwanne 17 dient zur Sammlung des an der Reaktionsschlange 14 und der Trenneinrichtung 15 gebildeten Kondensats. Ein Heizlüfter 18 hält die Temperatur im Geräteschrank der Einrichtung 1 auf über etwa 10°C.

Der Trenneinrichtung 15 ist ein Flüssigkeitswächter 19 nachgeschaltet, der das Meßgerät dann abschaltet, wenn ein Tropfenmitriß im Gas festgestellt wird.

Das von der Flüssigkeit getrennte, mit elementaren Schwermetallen bzw. den Hydriden beladene Gas gelangt sodann über eine Förderstrecke, die durch einen beheizten PTFE-Schlauch 20 gebildet wird, zum Analysengerät 2, in dem die Bestimmung des Gehaltes an Hg, As, Sb und Bi nach einer der bekannten Methoden erfolgt.

Die Eichung des Meßgerätes und gleichzeitige Funktionsprüfung kann nach einer der beiden folgenden Methoden vorgenommen werden:
- Anstelle der 1n HCl wird 1n HCl mit genau bekanntem Gehalt an Hg, As, Sb, Bi (zum Beispiel Hg ca. 2 mg/l) zudosiert, wobei statt Rauchgas Luft durch das Gerät gesaugt wird. Aus dem bekannten (und konstanten) Verhältnis Gas/1n HCl errechnet sich der Sollwert (zum Beispiel ca. 400 mg Hg/Nm³_{trocken}).
- Vor dem Ofen (wobei wiederum Luft statt Rauchgas angesaugt wird) werden ca. 20 ml/Std. einer Lösung von ca. 4 mg HgCl₂ und ca. 4 mg As³⁺/l in 1n HCl verdampft. Eine hinreichend konstante und spontante Verdampfung (unter Vermeidung von fraktionierter Verdampfung) gelingt in einer Quarzkapillare oder in einem PTFE-Schlauch (1m Länge, 0,5 mm Innendurchmesser), der spiralförmig um einen auf 200°C thermo-statisierten Heizkörper aufgerollt ist. Diese Methode erfordert zusätzliche Einrichtungen (Verdampfer etc.) und gestattet nur die Eichung auf Hg/As (As verdampft als AsCl₃, nicht dagegen Sb und Bi). Sie hat jedoch den Vorteil, daß damit auch der Ofen überprüft wird (z.B. hinsichtlich der Dichtigkeit des Quarzrohres).

Nach dem erfindungsgemäßen Verfahren lassen sich erstmals neben Quecksilber auch Arsen, Antimon und Wismut einwandfrei kontinuierlich messen. Die Eichung des Gerätes ist hierbei sehr einfach.

## Patentansprüche

1. Verfahren zur kontinuierlichen Messung von Schwermetallen in Abgasen, insbesondere von Müllverbrennungsanlagen, wobei
a) ein aus dem Abgas abgezweigter Probengasstrom erhitzt wird,
b) dem Probengasstrom sodann ein flüssiges saures Medium sowie ein Reduktionsmittel zugesetzt werden,
c) das Gas-Flüssigkeitsgemisch auf eine Temperatur unterhalb der Raumtemperatur abgekühlt wird,
d) hiernach das Gas von der Flüssigkeit getrennt und auf eine Temperatur erwärmt wird, bei der einerseits keine Kondensatbildung erfolgt und andererseits keine Reaktionen der Schwermetalle mit anderen Stoffen, insbesondere mit Sauerstoff, eintreten,
e) und schließlich in einer Analysenstufe der Gehalt des Gases an Schwermetallen bestimmt wird,
gekennzeichnet durch folgende Merkmale:
f) dem auf mindestens 700°C erhitzten Probengasstrom wird zunächst nur das flüssige saure Medium zugeführt, wobei die Reaktionsstrecke, in der die Reaktion zwischen dem Probengasstrom und dem flüssigen sauren Medium erfolgt, bei einer Temperatur von 50 bis 100°C, vorzugsweise bei etwa 70°C, gehalten wird;
g) im Anschluß hieran erfolgt die Zugabe von Tetrahydroboranat als Reduktionsmittel, wobei die Reaktionsstrecke, in der die Reaktion zwischen dem Reduktionsmittel und dem Gas-Flüssigkeitsgemisch erfolgt, auf einer Temperatur zwischen 5 und 10°C gehalten wird.

2. Verfahren nach Anspruch 1, dadurch gekennzeichnet, daß der Probengasstrom auf eine Temperatur von etwa 800°C erhitzt wird.

3. Verfahren nach Anspruch 1, dadurch gekennzeichnet, daß dem Probengasstrom als flüssiges saures Medium Salzsäure oder Salpetersäure in einer Konzentraion von 0,1 - 5n, vorzugsweise 1n zugeführt wird.

4. Verfahren nach Anspruch 1, dadurch gekennzeichnet, daß als Reduktionsmittel NaBH₄ zugesetzt wird.

5. Verfahren nach Anspruch 1, dadurch gekennzeichnet, daß das von der Flüssigkeit getrennte Gas auf eine Temperatur zwischen 20 und 60°C, vorzugsweise auf etwa 40°C, erwärmt wird.

6. Meßgerät zu kontinuierlichen Messung von Schwermetallen in Abgasen, insbesondere von Müllverbrennungsanlagen,
gekennzeichnet durch folgende Bauteile:
a) einen Ofen (5) zur Erhitzung eines aus dem Abgas abgezweigten Probengasstromes,
b) eine Dosiereinrichtung (8) zur dosierten Einführung eines flüssigen sauren Mediums in den Probengasstrom,
c) eine der Dosiereinrichtung (8) gemäß b) nachgeschaltete, auf einer Temperatur zwischen 50 und 100°C gehaltene erste Reaktionsschlange (10),
d) eine Dosiereinrichtung (11) zur dosierten Einführung von Tetrahydroboranat in das Gas-Flüssigkeitsgemisch,
e) eine der Dosiereinrichtung (11) gemäß d) nachgeschaltete, auf einer Temperatur zwischen 5 und 10°C gehaltene zweite Reaktionsschlange (14),
f) eine Trenneinrichtung (15) zur Trennung des Gases von der Flüssigkeit,
g) eine der Trenneinrichtung (15) gemäß f) nachgeschaltete, auf einer Temperatur zwischen 20 und 60°C gehaltene Förderstrecke (Schlauch 20) zum Transport des mit Schwermetallen beladenen Gases,
h) ein an die Förderstrecke gemäß g) angeschlossenes Analysengerät (2) zur Messung des Gasgehaltes an Schwermetallen.

7. Meßgerät nach Anspruch 6, dadurch gekennzeichnet, daß zwischen der Trenneinrichtung (15) und der Förderstrecke ein Flüssigkeitswächter (19) zur Abschaltung des Meßgerätes bei Tropfenmitriß im Gas vorgesehen ist.

8. Meßgerät nach Anspruch 6, dadurch gekennzeichnet, daß alle mit dem Gas in Berührung kommenden Teile aus Quarzglas oder PTFE bestehen.

## Claims

1. Method of continuous measurement of heavy metals in exhaust gases, particularly from refuse incineration plants, in which
a) a sample gas stream branched off from the exhaust gas is heated,
b) a liquid acid medium and a reducing agent are then added to the sample gas stream,
c) the gas/liquid mixture is cooled to a temperature below room temperature,
d) after this the gas is separated from the liquid and heated to a temperature at which on the one hand no condensate is formed and on the other hand no reactions of the heavy metals with other substances, particularly with oxygen, occur,
e) and finally, the heavy metal content of the gas is determined in an analysis stage,
characterised by the following features:
a) at first only the liquid acid medium is delivered to the sample gas stream, which has been heated to at least 700°C, and the reaction stage in which the reaction between the sample gas stream and the liquid acid medium takes place, is kept at a temperature of 50 to 100°C, preferably approximately 70°C;
b) after this tetrahydroborate is added as reducing agent, and the reaction stage in which the reaction between the reducing agent and the gas/liquid mixture takes place is kept at a temperature between 5 and 10°C.

2. Method as claimed in Claim 1, characterised in that the sample gas stream is heated to a temperature of approximately 800°C.

3. Method as claimed in Claim 1, characterised in that hydrochloric acid or nitric acid is delivered to the sample gas stream as liquid acid medium in a concentration of 0.1 to 5n, preferably 1n.

4. Method as claimed in Claim 1, characterised in that NaBH₄ is added as reducing agent.

5. Method as claimed in Claim 1, characterised in that the gas separated from the liquid is heated to a temperature between 20 and 60°C, preferably to approximately 40°C.

6. Measuring device for continuous measurement of heavy metals in exhaust gases, particularly from refuse incineration plants, characterised by the following components:
a) a furnace (5) for heating a sample gas stream branched off from the exhaust gas,
b) a dosaging arrangement (8) for dosaged introduction of a liquid acid medium into the sample gas stream,
c) a first reaction coil (10) which is arranged after the dosaging arrangement (8) according to b) and is kept at a temperature between 50 and 100°C,
d) a dosaging arrangement (11) for dosaged introduction of tetrahydroborate into the gas/liquid mixture,
e) a second reaction coil (14) which is arranged after the dosaging arrangement (11) according to d) and is kept at a temperature between 5 and 10°C,
f) a separating arrangement (15) for separating the gas from the liquid,
g) a conveyor stage (hose 20) which is arranged after the separating arrangement (15) according to f) and kept at a temperature between 20 and 60°C for transporting the gas charged with heavy metals,
h) an analysis device (2) connected to the conveyor stage according to g) for measuring the heavy metal content of the gas.

7. Measuring device as claimed in Claim 6, characterised in that a fluid monitor (19) is provided between the separating arrangement (15) and the conveyor stage to switch off the measuring device if drops are entrained in the gas.

8. Measuring device as claimed in Claim 6, characterised in that all the parts coming into contact with the gas are made from quartz glass or PTFE.

## Revendications

1. Procédé de mesure en continu de la teneur en métaux lourds de gaz résiduaires, en particulier d'installations d'incinération d'ordures, dans lequel :
a) un courant de gaz échantillon prélevé sur le gaz résiduaire est chauffé,
b) un milieu acide liquide ainsi qu'un agent réducteur sont additionnés ensuite au courant de gaz échantillon,
c) le mélange de gaz et de liquide est refroidi à une température inférieure à la température ambiante,
d) ensuite le gaz est séparé du liquide et chauffé à une température à laquelle d'une part aucune formation de condensat n'a lieu et d'autre part aucune réaction des métaux lourds avec d'autres substances, en particulier avec l'oxygène, ne se produit,
e) et finalement la teneur du gaz en métaux lourds se détermine dans un étage d'analyse,
caractérisé par les particularités suivantes :
f) tout d'abord seul le milieu acide liquide est additionné au courant de gaz échantillon chauffé à au moins 700°C, le trajet de réaction sur lequel a lieu la réaction entre le courant de gaz échantillon et le milieu acide liquide étant maintenu à une température de 50 à 100°C, de préférence à environ 70°C ;
g) ensuite a lieu l'addition de tétrahydroborate constituant un agent réducteur, le trajet de réaction, sur lequel la réaction entre l'agent réducteur et le mélange de gaz et de liquide a lieu, étant maintenu à une température comprise entre 5 et 10°C.

2. Procédé selon la revendication 1, caractérisé en ce que le courant de gaz échantillon est chauffé à une température d'environ 800°C.

3. Procédé selon la revendication 1, caractérisé en ce que de l'acide chlorhydrique ou de l'acide nitrique en une concentration de 0,1-5n, de préférence de 1n, qui constitue le milieu acide liquide, est envoyé dans le courant de gaz échantillon.

4. Procédé selon la revendication 1, caractérisé en ce que l'agent réducteur qui est additionné est NaBH₄.

5. Procédé selon la revendication 1, caractérisé en ce que le gaz séparé du liquide est chauffé à une température comprise entre 20 et 60°C, de préférence à environ 40°C.

6. Appareil de mesure en continu de la teneur en métaux lourds de gaz résiduaires, en particulier d'installations d'incinération d'ordures, caractérisé par les composants suivants :
a) un four (5) de chauffage d'un courant de gaz échantillon prélevé sur le gaz résiduaire,
b) un dispositif doseur (8) d'introduction dosée d'un milieu acide liquide dans le courant de gaz échantillon,
c) un premier serpentin de réaction (10) monté en aval du dispositif doseur (8) selon b) et maintenu à une température comprise entre 50 et 100°C,
d) un dispositif doseur (11) d'introduction dosée de tétrahydroborate dans le mélange de gaz et de liquide,
e) un second serpentin de réaction (14) monté en aval du dispositif doseur (11) selon d) et maintenu à une température comprise entre 5 et 10°C,
f) un dispositif séparateur (15) de séparation du gaz et du liquide,
g) un trajet de transport (flexible 20) monté en aval du dispositif séparateur (15) selon f) et maintenu à une température comprise entre 20 et 60°C pour le transport du gaz chargé de métaux lourds,
h) un appareil d'analyse (2) raccordé au trajet de transport selon g) pour la mesure de la teneur du gaz en métaux lourds.

7. Appareil de mesure selon la revendication 6, caractérisé en ce qu'un contrôleur de liquide (19) pour l'arrêt de l'appareil de mesure en cas d'entraînement de gouttelettes dans le gaz est prévu entre le dispositif séparateur (15) et le trajet de transport.

8. Appareil de mesure selon la revendication 6, caractérisé en ce que toutes les pièces entrant en contact avec le gaz sont en verre quartzeux ou en PTFE.
